(19) Europäisches Patentamt
European Patent Office
Office européen des brevets

(11) EP 0 839 521 B1

(12) **FASCICULE DE BREVET EUROPEEN**

(45) Date de publication et mention de la délivrance du brevet: **07.01.1999 Bulletin 1999/01**

(51) Int. Cl.$^6$: **A61K 7/48**, A61K 7/50

(21) Numéro de dépôt: **97402367.3**

(22) Date de dépôt: **08.10.1997**

(54) **Composition moussante pour le nettoyage de la peau sous la forme d'un gel transparent**

Schäumende Zusammensetzung für die Reinigung der Haut in Form eines transparenten Gels

Foaming composition for the cleaning of the skin in the form of a transparent gel

(84) Etats contractants désignés:
**DE ES FR GB IT**

(30) Priorité: **04.11.1996 FR 9613405**

(43) Date de publication de la demande:
**06.05.1998 Bulletin 1998/19**

(73) Titulaire: **L'OREAL**
**75008 Paris (FR)**

(72) Inventeurs:
- **Gagnebien, Didier**
  **92320 Chatillon (FR)**
- **Simon, Pascal**
  **94400 Vitry/S/Seine (FR)**

(74) Mandataire:
**Tezier Herman, Béatrice**
**L'OREAL,**
**Département Propriété Industrielle,**
**90, rue du Gal Roguet**
**92583 Clichy Cédex (FR)**

(56) Documents cités:

| | |
|---|---|
| EP-A- 0 274 812 | EP-A- 0 588 379 |
| WO-A-92/07543 | WO-A-93/08840 |
| WO-A-94/17830 | DE-A- 4 139 935 |
| FR-A- 2 237 615 | US-A- 4 268 498 |
| US-A- 4 482 537 | US-A- 4 687 843 |

- **CHEMICAL ABSTRACTS, vol. 93, no. 18, 3 novembre 1980 Columbus, Ohio, US; abstract no. 173598z, page 345; colonne I; XP002029045 & COSMET.TOILETRIES, vol. 95, no. 3, 1980, pages 85-86,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 839 521 B1

## Description

L'invention a pour objet des compositions moussantes riches en huiles, d'aspect gélifié, transparentes, rinçables à l'eau, pour le nettoyage de la peau.

Le nettoyage de la peau est très important pour le soin du visage. Il doit être le plus performant possible car les résidus gras tels que l'excès de sébum, les restes des produits cosmétiques utilisés quotidiennement et les produits de maquillage, notamment les produits "waterproof', résistants à l'eau, s'accumulent dans les replis cutanés et à la surface de la peau et peuvent obstruer les pores de la peau, entraîner l'apparition de boutons. Une mauvaise qualité de nettoyage, et en particulier un mauvais rinçage, sont souvent responsables parmi d'autres facteurs de cause, d'un teint brouillé.

On connaît plusieurs grands types de produits de nettoyage de la peau : les lotions et les gels aqueux détergents moussants, et les huiles et les gels anhydres nettoyants rinçables, les laits démaquillants, les crèmes moussantes, qui sont habituellement à base de savon.

Les lotions et les gels aqueux détergents moussants ont une action nettoyante grâce aux tensioactifs qui mettent en suspension les résidus gras et les pigments des produits de maquillage. Ils sont efficaces et agréables cosmétiquement du fait qu'ils moussent et qu'ils sont facilement éliminés. Dans la mesure où ils ne contiennent pas d'huile cosmétique, ils présentent l'inconvénient d'assécher la peau par leur action délipidante. C'est le cas par exemple des produits décrits par le document WO95/05769, qui enseigne des lotions pour le nettoyage de la peau, très fluides, pressurisables, produisant une belle mousse, mais ces produits détruisent le film hydro-lipidique de la peau et laissent la peau propre mais rêche.

Les crèmes et les laits démaquillants contiennent à la fois des huiles, des émulsionnants et des tensioactifs détergents en quantité suffisamment faible pour ne pas déstabiliser l'émulsion. Ces produits en dépit de leur bonne efficacité ne sont pas moussants et présentent une rinçabilité insuffisante qui nécessite l'emploi d'une lotion tonique détergente complémentaire pour parfaire le rinçage et l'élimination des salissures. Outre son caractère astreignant, l'utilisation de ce deuxième produit peut entraîner à long terme un assèchement de la peau.

Dans le domaine du nettoyage de la peau, le démaquillage des produits "waterproof', résistants à l'eau, des produits sans transfert et des maquillages lourds, comme les maquillages de théâtre, pour être efficace, nécessite l'utilisation de composés huileux.

Les compositions huileuses sont reconnues pour leur efficacité comme nettoyant et/ou démaquillant. Elles permettent en effet de dissoudre très facilement les salissures lipophiles et le maquillage, en particulier les maquillages ⟨⟨waterproof⟩⟩ et sans transfert réputés difficiles à démaquiller. Ces produits sont efficaces et bien tolérés. Ils présentent l'inconvénient de ne pas mousser et de ne pas conférer de sensation de fraîcheur à l'application, ce qui est pénalisant d'un point de vue cosmétique. En outre, les compositions huileuses ayant l'aspect d'un gel sont épaissies habituellement par des cires, des silices, des argiles modifiées ou des sels polyvalents d'acides gras qui leur confèrent un aspect habituellement trouble ou opaque, peu attrayant ; leur stabilité dans le temps est souvent limitée.

On a cherché à concevoir des produits moussants nettoyants, parfaitement rinçables à l'eau, comprenant des huiles en quantités élevées de façon à rendre optimal le nettoyage de la peau et à hydrater et nourrir celle-ci en évitant tout phénomène d'assèchement et d'irritation. En outre, on a cherché à préparer des compositions ayant un aspect attractif, celui d'un gel transparent, ces compositions étant facilement éliminées par un rinçage à l'eau et étant parfaitement stables au stockage.

Pour réaliser un tel produit, on ne peut pas simplement introduire des huiles dans une lotion ou un gel aqueux. En effet, les huiles ont tendance à inhiber les propriétés moussantes de ces formulations ; on dit que les huiles ⟨⟨tuent⟩⟩ la mousse.

L'invention a pour objet des compositions pour le nettoyage de la peau ayant l'aspect de, ou comprenant, un gel moussant transparent stable. Ces compositions sont caractérisées par le fait qu'elles comprennent :

(i) une phase grasse,
(ii) au moins un ester gras d'hydrate de carbone en $C_5$-$C_7$,
(iii) au moins un polyol, et
(iv) au moins un tensioactif moussant.

Le mot ⟨⟨transparent⟩⟩ signifie qu'au travers d'une bouteille transparente contenant la composition on peut distinguer les caractères imprimés sur une page de journal placée derrière cette bouteille.

La viscosité des compositions selon l'invention est préférentiellement supérieure à 2 Pa.s encore plus préférentiellement à 4 Pa.s et encore plus préférentiellement à 5 Pa.s. De préférence elle est inférieure à 25 Pa.s. Cette viscosité est mesurée à la température ambiante (20 à 25°C) et à pression ambiante, au RHEOMAT 180 (METTLER) avec un corps de mesure à 200 tours/minute.

On connaissait dans l'art antérieur, par exemple par les documents JP-5-229916 et JP-60-115509, des compositions huileuses pour le nettoyage de la peau sous la forme d'un gel transparent stable comprenant un ester gras de saccharose hydrophile et un polyol, ces compositions pouvant comprendre un tensioactif moussant. Toutefois, ces compositions présentent plusieurs inconvénients : les dérivés de saccharose se caractérisent par une forte instabilité à la température ; les compositions décrites dans ces documents comprennent de faibles pourcentages de tensioactif moussant par rapport à leur pourcentage en huile, ce faible taux de moussant ne permettant pas la formation d'une mousse ; lorsque l'on augmente le pourcentage de tensioactif moussant dans ces compositions on obtient un mélange instable, opaque, déphasé, comme il sera montré dans les essais comparatifs.

Les compositions selon l'invention remédient aux inconvénients de l'art antérieur. Ces compositions et leurs propriétés étonnantes n'étaient ni mentionnées ni suggérées dans l'art antérieur.

De préférence ces compositions comprennent également de l'eau. Dans ce cas elles se présentent avantageusement sous la forme d'une émulsion huile-dans-eau ayant l'aspect d'un gel.

Par hydrates de carbone en $C_5$-$C_7$, on entend des pentoses, hexoses et heptoses (non réduits) et leurs dérivés alkyl-olosides dont le groupement alkyle contient de 1 à 6 atomes de carbone. Avantageusement, on choisit les hydrates de carbone parmi ceux ayant une chaîne à 6 atomes de carbone, et préférentiellement ils sont choisis parmi le glucose, le fructose et leurs dérivés alkyl-glucoside et alkyl-fructoside en $C_1$-$C_6$. Encore plus préférentiellement, l'hydrate de carbone est le glucose ou un dérivé alkyl-glucoside, comme par exemple le méthyl-1-glucoside.

Par esters gras d'hydrates de carbone en $C_5$-$C_7$, on entend de préférence les composés obtenus par réaction d'un acide gras à chaîne saturée ou insaturée ayant de 8 à 30 atomes de carbone, préférentiellement de 12 à 22 atomes de carbone et encore plus préférentiellement de 16 à 20 atomes de carbone avec un hydrate de carbone choisi parmi les pentoses, les hexoses, les heptoses et leurs dérivés alkyl-olosides dont le groupement alkyle contient de 1 à 6 atomes de carbone.

L'ester d'acide gras et d'hydrate de carbone peut contenir un mélange de dérivés mono-, di-, tri-, tétra-ester.

Avantageusement, les esters d'hydrates de carbone en $C_5$-$C_7$ sont oxyalkylénés. Préférentiellement les esters d'hydrates de carbone en $C_5$-$C_7$ sont éthérifiés par un ou plusieurs fragments oxyéthylène ou oxypropylène, l'ensemble des substituants oxyéthylène ou oxypropylène représentant au total de 5 à 200, et préférentiellement 15 à 150, unités oxyde d'alkylène.

Avantageusement toutes les fonctions hydroxyle des dérivés d'hydrate de carbone utilisés dans les compositions selon l'invention sont substituées par un groupement ester ou par un groupement oxyde d'alkylène.

De tels composés sont bien connus de l'homme du métier. Plusieurs de ces composés sont disponibles commercialement. Par exemple, on peut utiliser les dérivés vendus sous la marque GLUCAMATE par la société AMERCHOL.

Avantageusement les esters d'hydrate de carbone en $C_6$ sont choisis parmi les composés répondant à la formule (I) :

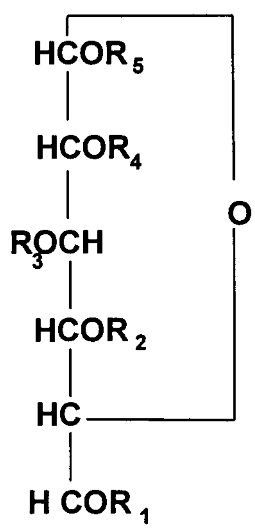

(I)

dans laquelle

$R_1$ est un groupement de formule (III) :

$$-OC-R' \qquad (III)$$

R' étant choisi parmi les groupements alkyle linéaires ou ramifiés, saturés ou insaturés en $C_8$-$C_{30}$ et préférentiellement en $C_{12}$-$C_{22}$,

$R_2$, $R_3$, $R_4$, identiques ou différents, représentent un groupement choisi parmi :

- un atome d'hydrogène,
- un groupement de formule (II) :

$$-(C_nH_{2n}O)_p-R \qquad (II)$$

avec n=2 ou 3 ; p est un entier allant de 2 à 50; R représente un atome d'hydrogène ou un groupement alkyle en $C_1$-$C_6$,
- un groupement de formule (III) :

$$-OC-R' \qquad (III)$$

R' étant choisi parmi les groupements alkyle linéaires ou ramifiés, saturés ou insaturés en $C_8$-$C_{30}$ et préférentiellement en $C_{12}$-$C_{22}$,
l'un au moins de $R_2$, $R_3$, $R_4$ étant un groupement de formule (II), le total des résidus oxyalkylénés, $\Sigma p$, étant compris entre 5 et 200,

$R_5$ étant un groupement alkyle en $C_1$-$C_6$ linéaire ou ramifié, saturé ou insaturé.

De préférence, on choisit :

- $R_5 = CH_3$,
- au moins deux groupements parmi $R_2$, $R_3$, $R_4$, répondent à la formule (II),
- $\Sigma p$ est compris entre 15 et 150,
- R=H,
- au moins un groupement parmi $R_1$, $R_2$, $R_3$, $R_4$, répond à la formule (III),
- R' est choisi parmi les groupements alkyle en $C_{16}$-$C_{20}$,
- aucun des groupements $R_2$, $R_3$, $R_4$, n'est l'atome d'hydrogène.

Avantageusement, les compositions selon l'invention comprennent, en poids par rapport au poids total de la composition, de 0,5 à 50% d'ester d'hydrate de carbone en $C_5$-$C_7$ et préférentiellement de 2 à 20%.

Les compositions selon l'invention comprennent au moins un polyol. Ce polyol peut éventuellement être oxyalkyléné. Avantageusement, le polyol comprend au moins deux fonctions hydroxyle libres. Ce polyol peut être choisi parmi l'éthylène glycol, le propylène glycol, le 1,3-butylène glycol, le dipropylène glycol, la glycérine, les polyglycérines, comme la diglycérine, la triglycérine et la tétraglycérine, le glucose, le maltose, le maltitol, le saccharose, le fructose, le sorbitol, les sucres issus de la décomposition de l'amidon et leurs mélanges.

Le polyol représente avantageusement de 0,5 à 60% en poids par rapport au poids total de la composition, de préférence de 2 à 40% et encore plus préférentiellement de 5 à 30%.

La nature de la phase grasse rentrant dans la composition des émulsions selon l'invention n'est pas critique; elle peut ainsi être constituée par tous les composés qui sont déjà connus de façon générale comme convenant pour la fabrication d'émulsions de type huile-dans-eau. En particulier, ces composés peuvent être choisis, seuls ou en mélanges, parmi les différents corps gras, les huiles d'origine végétale, animale ou minérale, les cires naturelles ou synthétiques, et analogues.

On peut citer parmi les huiles utilisables dans la présente invention les huiles d'origine végétale ou animale, comme par exemple le squalane, l'huile de coprah, l'huile de macadamia, l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ; des huiles d'hydrocarbures telles que des huiles de paraffine, la vaseline, l'isododécane, l'isohexadécane, les isoparaffines ; des huiles de silicone comme les polydiméthylsiloxanes, les cyclopolydiméthylsiloxanes les polyméthylphénylsiloxanes, des polysiloxanes modifiés par des acides gras, des polysiloxanes modifiés par des alcools gras, des polysiloxanes modifiés par des polyoxyalkylènes, des silicones fluorées; des huiles perfluorées et/ou organofluorées; des acides gras supérieurs tels que l'acide myristique, l'acide palmitique, l'acide stéarique, l'acide béhénique, l'acide oléique, l'acide linoléique, l'acide linolénique, l'acide isostéarique, des alcools gras supérieurs tels que le cétanol, l'alcool stéarylique, l'alcool oléique; des mono et des di-esters parmi lesquels on peut citer en particulier le myristate d'isopropyle, le palmitate d'isopropyle, le stéarate

de butyle, le laurate d'hexyle, l'isononanoate d'isononyle, le palmitate de 2-éthyl hexyle, le laurate de 2-hexyl décyle, le palmitate de 2-octyl décyle, le myristate de 2-octyl dodécyle, le succinate de 2-diéthylhexyle, le malate de diisostéaryle, le lactate de 2-octyl dodécyle, le triisostéarate de glycérine, l'adipate de di-n-butyle, l'adipate de di-(2-éthyl hexyle), le dioléate d'éthylène glycol, le di-isotridécanoate d'éthylène glycol, le di-isostéarate d'éthylène glycol, le di-caprylate de néopentylglycol.

De préférence on utilise une huile ou un mélange d'huiles ayant un indice de réfraction $^{20}n_D \leq 1{,}45$.

La phase grasse peut représenter de 1 à 95% en poids du poids total de la composition, de préférence de 20 à 85% et encore plus préférentiellement de 40 à 80%.

Les compositions selon l'invention comprennent au moins un tensioactif moussant. On peut se reporter au document ⟨⟨Encyclopedia of Chemical Technology, KIRK-OTHMER⟩⟩, volume 22, p.333-432, 3ème édition, 1979, WILEY, où sont citées les principales classes de tensioactifs connues de l'homme du métier ainsi que leurs fonctions (en particulier le fait d'être moussant).

Les tensioactifs moussants utilisés selon la présente invention sont choisis parmi les tensioactifs possédant un pouvoir moussant caractérisé par une hauteur de mousse supérieure à 100 mm ; de préférence, supérieure à 120 mm mesurée selon la méthode ROSS-MILES pour une solution à 0,1 % en poids de tensioactif dans de l'eau distillée à 25° C.

Préférentiellement, ces tensioactifs sont choisis parmi les tensioactifs anioniques et les tensioactifs amphotères. Parmi ces classes de tensioactifs, on peut citer :

- tensioactifs amphotères : les alkylbétaïnes, les alkylsulfobétaïnes, les alkylamidopropylbétaïnes, les alkylcarboxyglycinates de métal alcalin ou alcalinoterreux, les imidazolines ; parmi ces familles de tensioactifs, on peut citer plus particulièrement les exemples suivants : la diméthyl bétaïne, la coco bétaïne, la coco-amidopropylbétaïne, la coco-amidopropyl hydroxysultaïne, le di-sodium cocoamphodiacétate ; - tensioactifs anioniques : les alkyl phosphates, comme par exemple le lauryl phosphate de sodium, les alkyl taurates, comme par exemple le méthyl palmitoyl taurate de sodium, les sulfosuccinates, comme par exemple le sulfosuccinate de cocoyl, le sel disodique du sulfosuccinate d'alcool laurique oxyéthyléné, les alkyl sulfates, comme par exemple le lauryl sulfate de triéthanolamine, les sarcosinates, comme par exemple le lauroyl sarcosinate de sodium, les alkyl éthersulfates, comme par exemple le lauroyl éther sulfate de sodium, les iséthionates, comme par exemple le cocoyl isethionate de sodium, les alkyléther carboxylates, comme par exemple le décyl éther carboxylate de sodium oxyéthyléné.

On peut avantageusement prévoir d'utiliser dans les compositions selon l'invention un tensioactif moussant secondaire non-ionique pour améliorer les qualités de la mousse (densité onctuosité, douceur). Ce moussant peut par exemple être choisi parmi :

- les composés de formule :

$$R^1 O(G)_p \qquad (II)$$

  dans laquelle $R^1$ est une chaîne hydrocarbonée monovalent contenant de 1 à 30 atomes de carbone, G est un dérivé de saccharide contenant de 5 à 6 atomes de carbone et p est une valeur moyenne statistique allant de 1 à 6 ; les composés de formule (II) sont décrits notamment dans la demande WO 94/27562. Les composés préférentiels sont ceux pour lesquels le radical $R^1$ est un radical alkyle en $C_8$-$C_{16}$ et plus particulièrement ceux dont le degré de polymérisation est 1,4 ou 1,6 ; on peut citer par exemple :

  . les alkylpolyglycosides commercialisés par HENKEL sous les noms APG 225, APG 425, APG 625 ;
  . les alkylpolyglucosides commercialisés par HENKEL sous le nom GLUCOPON proche de l'APG 625 avec un degré de polymérisation différent ;
  . les alkylpolyglycosides commercialisés par HENKEL sous le nom PLANTAREN tels que le PLANTAREN 2000 qui est un $C_8$-$C_{16}$-alkyl-polyglucoside avec un degré de polymérisation de 1,4 ; le PLANTAREN 1200 et qui est un $C_{12}$-$C_{18}$-alkyl-polyglucoside de degré de polymérisation 1,4 ; le PLANTAREN 1300, qui est un $C_{12}$-$C_{18}$-alkyl-polyglycoside de degré de polymérisation 1,6.

- les esters d'acide gras et de sorbitane oxyéthylénés, comme par exemple le produit commercialisé sous la marque TWEEN 20 par la société ICI.

Les tensioactifs moussants peuvent être présents à raison de 0,5 à 50%, de préférence de 2 à 40% et encore plus préférentiellement de 8 à 20% en poids par rapport au poids total de la composition.

L'eau représente préférentiellement de 0,01 à 30% en poids par rapport au poids total de la composition. Avantageusement l'eau représente de 2 à 20% en poids par rapport au poids total de la composition.

Habituellement on entend par eau de l'eau pure. Toutefois, une partie de l'eau utilisée dans les compositions selon l'invention peut éventuellement être choisie parmi les eaux minérales ou thermales. En général, une eau minérale est propre à la consommation, ce qui n'est pas toujours le cas d'une eau thermale. Chacune de ces eaux contient, entre autre, des minéraux solubilisés et des oligo-éléments. Ces eaux sont connues pour être employées à des fins de traitement spécifique selon les oligo-éléments et les minéraux particuliers qu'elles contiennent, tels que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou oligo-élémentaires préparées à partir d'eau purifiée (déminéralisée ou distillée).

Une eau thermale ou minérale naturelle utilisée selon l'invention peut, par exemple être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Uriage-les-bains, l'eau d'Avene.

Les compositions cosmétiques de l'invention peuvent, en outre, contenir des adjuvants hydrosolubles ou liposolubles habituels dans le domaine cosmétique tels que les conservateurs, les antioxydants, les parfums, les filtres, les matières colorantes, les nacres, les actifs hydrophiles ou lipophiles.

Elles peuvent également contenir des charges insolubles: poudre de polyéthylène, particules de polyamide comme par exemple celles vendues sous la dénomination "ORGASOL" par la Société ATOCHEM également connues sous les noms (CTFA) de *"polyamide 12"* ou *"polyamide* 6". On peut également utiliser dans ces compositions du kaolin, des poudres de Nylon répertoriées sous le nom CTFA de *"Nylon 12"* ou *"Nylon 6".* De telles compositions sont intéressantes dans le nettoyage de peau pour leurs propriétés exfoliantes.

Les compositions selon l'invention ont l'apparence d'un gel, transparent, stable. En outre ces compositions ont une très bonne rinçabilité. En particulier, les esters gras d'hydrates de carbone en $C_5$ à $C_7$ confèrent à ces compositions une rinçabilité améliorée par rapport aux compositions à base d'esters de saccharose et celles à base d'esters de sorbitol.

Les compositions selon l'invention peuvent se présenter par exemple sous la forme d'un produit nettoyant, démaquillant, d'un produit hydratant, d'un produit de gommage, d'un exfoliant.

**EXEMPLES :**

Pour chaque composition, les pourcentages sont donnés en poids de matière active par rapport au poids total de la composition.

La viscosité est mesurée à 25°C à l'aide d'un viscosimètre Rhéomat 180, les mesures de viscosité sont données en Pascal seconde (Pa.s).

Exemple 1 : Composition nettoyante corps et visage :

| | |
|---|---|
| Phase aqueuse : | |
| Sesqui-stéarate de méthyl-glucose oxyéthyléné (20OE) | 4,5 % |
| Glycérol | 9 % |
| Eau | 4,5 % |
| Phase huileuse : | |
| Huile de vaseline | 9 % |
| Palmitate d'éthyl-2-hexyle | 13 % |
| Isoparaffine hydrogénée | 8 % |
| Cyclopentadiméthylsiloxane | 16 % |
| Isododécane | 20 % |
| Parfum | qs % |
| Conservateurs | qs % |
| Tensioactifs | |
| Lauryl éther sulfate de sodium | 8 % |
| Mono laurate de sorbitane oxyéthyléné (20OE) | 5 % |

Mode opératoire : le dérivé de glucose est dissout dans l'eau et les polyols sous agitation entre 25°C et 35°C jusqu'à l'obtention d'une pâte visqueuse homogène. La phase huileuse puis les tensioactifs sont alors introduits lentement sous forte agitation. Le produit est ensuite lissé la pâle et débullé sous vide.

Cette composition a un aspect gélifié, transparent, cristallin, une viscosité Rhéomat égale à 20 Pa.s. Ce produit, qui s'utilise sous la douche, permet un nettoyage efficace en douceur. Il laisse la peau lisse et nette sans résidu ou film huileux.

Exemple 2 : Composition démaquillante :

| | |
|---|---|
| Phase aqueuse : | |
| Sesqui-stéarate de méthyl-glucose oxyéthyléné (20OE) | 0,8 % |
| Di-oléate de méthyl glucose oxyéthyléné (120OE) | 2,5 % |
| Glycérol | 13 % |
| Eau | 3,5 % |
| Phase huileuse : | |
| Huile de vaseline | 8 % |
| Palmitate d'éthyl-2-hexyle | 22 % |
| Isoparaffine hydrogénée | 7 % |
| Cyclopentadiméthylsiloxane | 11 % |
| Isododécane | 16,5% |
| Parfum | qs |
| Conservateurs | qs |
| Tensioactifs : | |
| Cocoamphodiacétate | 10 % |
| Sodium lauroyl sarcosinate | 5 % |

Cette composition a un aspect gélifié, transparent, cristallin, une viscosité Rhéomat égale à 12 Pa.s. L'étalement du produit sur la peau est facile, la rinçabilité excellente. Après rinçage, la peau est nette, d'un toucher soyeux, non gras.

**ESSAIS COMPARATIFS :**

Afin de montrer l'intérêt des compositions selon l'invention par rapport aux compositions de l'art antérieur, on a réalisé les essais suivants :

Essais 1 : on a reproduit la composition de l'exemple 1 ci-dessus en remplaçant le sesqui-stéarate de méthyl-glucose oxyéthyléné (20OE) par du palmito-stéarate de saccharose (même pourcentage en poids). On obtient une composition instable, qui se sépare en deux phases après 24 heures de stockage.
Essais 2 : on a reproduit la composition de l'exemple 2 ci-dessus en remplaçant le sesqui-stéarate de méthyl-glucose oxyéthyléné (20OE) et le di-oléate de méthyl glucose oxyéthyléné (120OE) par du palmito-stéarate de saccharose (même pourcentage en poids). On obtient une composition fluide et opaque.

**Revendications**

**1.** Composition pour le nettoyage de la peau ayant l'aspect de, ou comprenant, un gel moussant, transparent, caractérisée par le fait qu'elle comprend :

(i) une phase grasse,
(ii) au moins un ester gras d'hydrate de carbone en $C_5$-$C_7$,
(iii) au moins un polyol, et
(iv) au moins un tensioactif moussant.

**2.** Composition selon la revendication précédente, caractérisée en ce qu'elle comprend également de l'eau.

**3.** Composition selon l'une quelconque des revendications précédentes, caractérisée en ce que l'hydrate de carbone

en $C_5$-$C_7$ est choisi parmi les pentoses, hexoses et heptoses et leurs dérivés alkyl-oloside dont le groupement alkyle contient de 1 à 6 atomes de carbone.

**4.** Composition selon l'une quelconque des revendications précédentes, caractérisée en ce que l'hydrate de carbone est le méthyl-1-glucoside.

**5.** Composition selon l'une quelconque des revendications précédentes, caractérisée en ce que l'ester gras d'hydrate de carbone en $C_5$-$C_7$ est un composé obtenu par réaction d'un acide gras à chaîne saturée ou insaturée ayant de 8 à 30 atomes de carbone et de préférence 12 à 22 atomes de carbone avec l'hydrate de carbone.

**6.** Composition selon l'une quelconque des revendications précédentes, caractérisée en ce que l'ester gras d'hydrate de carbone en $C_5$-$C_7$ est oxyalkyléné.

**7.** Composition selon l'une quelconque des revendications précédentes, caractérisée en ce que l'ester d'hydrate de carbone en $C_5$-$C_7$ est éthérifié par un ou plusieurs fragments oxyéthylène ou oxypropylène, l'ensemble des substituants oxyéthylène ou oxypropylène représentant de 5 à 200 unités oxyde d'alkylène.

**8.** Composition selon l'une quelconque des revendications précédentes, caractérisée en ce que l'ester d'hydrate de carbone est choisi parmi les composés répondant à la formule (I) :

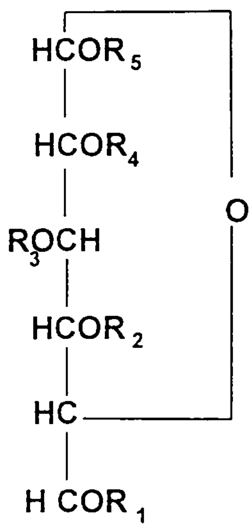

(I)

dans laquelle

$R_1$ est un groupement de formule (III) :

-OC-R'

R' étant choisi parmi les groupements alkyle linéaires ou ramifiés, saturés ou insaturés en $C_8$-$C_{30}$ et préférentiellement en $C_{12}$-$C_{22}$,

$R_2$, $R_3$, $R_4$, identiques ou différents, représentent un groupement choisi parmi :

- un atome d'hydrogène,
- un groupement de formule (II) :

$$-(C_nH_{2n}O)_p-R$$

avec n=2 ou 3 ; p est un entier allant de 2 à 50; R représente un atome d'hydrogène ou un groupement alkyle en $C_1$-$C_6$,
- un groupement de formule (III) :

-OC-R'

R' étant choisi parmi les groupements alkyle linéaires ou ramifiés, saturés ou insaturés en $C_8$-$C_{30}$ et préférentiellement en $C_{12}$-$C_{22}$,
l'un au moins de $R_2$, $R_3$, $R_4$ étant un groupement de formule (II), le total des résidus oxyalkylénés, $\Sigma p$, étant compris entre 5 et 200,

$R_5$ étant un groupement alkyle en $C_1$-$C_6$, linéaire ou ramifié, saturé ou insaturé.

**9.** Composition selon la revendication précédente, caractérisée en ce que

- $R_5 = CH_3$,
- au moins deux groupements parmi $R_2$, $R_3$, $R_4$, répondent à la formule (II),
- $\Sigma p$ est compris entre 15 et 150,
- R=H,
- au moins un groupement parmi $R_1$, $R_2$, $R_3$, $R_4$, répond à la formule (III),
- R' est choisi parmi les groupements alkyle en $C_{16}$-$C_{20}$,
- aucun des groupements $R_2$, $R_3$, $R_4$, n'est l'atome d'hydrogène.

**10.** Composition selon l'une quelconque des revendications précédentes, caractérisée en ce qu'elle comprend, en poids par rapport au poids total de la composition, de 0,5 à 50% d'ester d'hydrate de carbone en $C_5$-$C_7$ et préférentiellement de 2 à 20%.

**11.** Composition selon l'une quelconque des revendications précédentes, caractérisée en ce que le polyol est choisi parmi l'éthylène glycol, le propylène glycol, le 1,3-butylène glycol, le dipropylène glycol, la glycérine, la diglycérine, la triglycérine et la tétraglycérine, le glucose, le maltose, le maltitol, le saccharose, le fructose, le sorbitol, les sucres issus de la décomposition de l'amidon et leurs mélanges.

**12.** Composition selon l'une quelconque des revendications précédentes, caractérisée en ce qu'elle comprend de 0,5 à 60% de préférence de 2 à 40% et encore plus préférentiellement de 5 à 30%, en poids par rapport au poids total de la composition, de polyol.

**13.** Composition selon l'une quelconque des revendications précédentes, caractérisée en ce que la phase grasse comprend une huile ou un mélange d'huiles ayant un indice de réfraction $^{20}n_D \leq 1,45$.

**14.** Composition selon l'une quelconque des revendications précédentes, caractérisée en ce que la phase grasse représente de 1 à 95%, de préférence de 20 à 85%, en poids du poids total de la composition.

**15.** Composition selon l'une quelconque des revendications précédentes, caractérisée en ce que le tensioactif moussant est choisi parmi les tensioactifs possédant un pouvoir moussant caractérisé par une hauteur de mousse supérieure à 100 mm ; de préférence, supérieure à 120 mm mesurée selon la méthode ROSS-MILES pour une solution à 0,1 % en poids de tensioactif dans de l'eau distillée à 25° C.

**16.** Composition selon l'une quelconque des revendications précédentes, caractérisée en ce que le tensioactif moussant est choisi parmi les tensioactifs anioniques et les tensioactifs amphotères.

**17.** Composition selon l'une quelconque des revendications précédentes, caractérisée en ce que le tensioactif moussant est présent en quantité allant de 0,5 à 50 % en poids, de préférence 2 à 40 % en poids par rapport au poids total de la composition.

**18.** Composition selon l'une quelconque des revendications 2 à 17, caractérisée en ce que l'eau représente de 0,01 à 30%, avantageusement de 2 à 20%, en poids par rapport au poids total de la composition.

**19.** Composition selon l'une quelconque des revendications 2 à 18, caractérisée en ce que l'eau est choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Uriage-les-bains, l'eau d'Avene.

**20.** Composition selon l'une quelconque des revendications précédentes, caractérisée en ce qu'elle comprend en outre au moins une charge insoluble choisie parmi le kaolin, les poudre de polyéthylène, les particules de polyamide et les poudres de Nylon.

**21.** Composition selon l'une quelconque des revendications précédentes, caractérisée en ce qu'elle est sous la forme d'un produit nettoyant, démaquillant, d'un produit hydratant, d'un produit de gommage, d'un exfoliant.

## Claims

**1.** Composition for cleaning the skin, which has the appearance of, or includes, a foaming transparent gel, characterized in that it includes:

(i) a fatty phase,
(ii) at least one $C_5$-$C_7$ carbohydrate fatty ester,
(iii) at least one polyol, and
(iv) at least one foaming surfactant.

**2.** Composition according to the preceding claim, characterized in that it also includes water.

**3.** Composition according to either of the preceding claims, characterized in that the $C_5$-$C_7$ carbohydrate is chosen from pentoses, hexoses and heptoses and their alkyl holoside derivatives in which the alkyl group contains from 1 to 6 carbon atoms.

**4.** Composition according to any one of the preceding claims, characterized in that the carbohydrate is 1-methylglucoside.

**5.** Composition according to any one of the preceding claims, characterized in that the $C_5$-$C_7$ carbohydrate fatty ester is a compound obtained by reaction of a fatty acid containing a saturated or unsaturated chain containing from 8 to 30 carbon atoms and preferably 12 to 22 carbon atoms with the carbohydrate.

**6.** Composition according to any one of the preceding claims, characterized in that the $C_5$-$C_7$ carbohydrate fatty ester is oxyalkylenated.

**7.** Composition according to any one of the preceding claims, characterized in that the $C_5$-$C_7$ carbohydrate ester is etherified by one or more oxyethylene or oxypropylene fragments, the total of the oxyethylene or oxypropylene substituents representing from 5 to 200 alkylene oxide units.

**8.** Composition according to any one of the preceding claims, characterized in that the carbohydrate ester is chosen from the compounds corresponding to the formula (I):

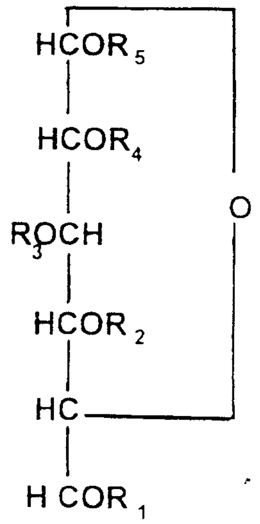

(I)

in which

$R_1$ is a group of formula (III):

$$-OCR' \qquad (III)$$

R' being chosen from $C_8$-$C_{30}$ and preferably $C_{12}$-$C_{22}$ saturated or unsaturated, linear or branched alkyl groups,

$R_2$, $R_3$ and $R_4$, which are identical or different, denote a group chosen from:

- a hydrogen atom,
- a group of formula (II):

$$-(C_nH_{2n}O)_p-R \qquad (II)$$

with n = 2 or 3; p is an integer ranging from 2 to 50; R denotes a hydrogen atom or a $C_1$-$C_6$ alkyl group,
- a group of formula (III):

$$-OC-R' \qquad (III)$$

R' being chosen from $C_8$-$C_{30}$ and preferably $C_{12}$-$C_{22}$ saturated or unsaturated, linear or branched alkyl groups,
at least one of $R_2$, $R_3$ and $R_4$ being a group of formula (II), the total of the oxyalkylene residues, $\Sigma p$, being between 5 and 200,

$R_5$ being a saturated or unsaturated, linear or branched $C_1$-$C_6$ alkyl group.

**9.** Composition according to the preceding claim, characterized in that

- $R_5 = CH_3$,
- at least two groups from $R_2$, $R_3$ and $R_4$ correspond to the formula (II),
- $\Sigma p$ is between 15 and 150,
- R = H,
- at least one group from $R_1$, $R_2$, $R_3$ and $R_4$ corresponds to the formula (III),
- R' is chosen from $C_{16}$-$C_{20}$ alkyl groups,
- none of the groups $R_2$, $R_3$ and $R_4$ is the hydrogen atom.

10. Composition according to any one of the preceding claims, characterized in that it includes, by weight relative to the total weight of the composition, from 0.5 to 50 % of $C_5$-$C_7$ carbohydrate ester, and preferably from 2 to 20 %.

11. Composition according to any one of the preceding claims, characterized in that the polyol is chosen from ethylene glycol, propylene glycol, 1,3-butylene glycol, dipropylene glycol, glycerine, diglycerine, triglycerine and tetraglycerine, glucose, maltose, maltitol, sucrose, fructose, sorbitol, sugars originating from the decomposition of starch and mixtures thereof.

12. Composition according to any one of the preceding claims, characterized in that it includes from 0.5 to 60 %, preferably from 2 to 40 % and still more preferably from 5 to 30 %, by weight relative to the total weight of the composition, of polyol.

13. Composition according to any one of the preceding claims, characterized in that the fatty phase includes an oil or a mixture of oils which has a refractive index $n_D^{20} \leq 1.45$.

14. Composition according to any one of the preceding claims, characterized in that the fatty phase represents from 1 to 95 %, preferably from 20 to 85 %, by weight of the total weight of the composition.

15. Composition according to any one of the preceding claims, characterized in that the foaming surfactant is chosen from surfactants which have a foaming power characterized by a foam height greater than 100 mm, preferably greater than 120 mm, measured according to the Ross-Miles method for a solution containing 0.1 % by weight of surfactant in distilled water at 25°C.

16. Composition according to any one of the preceding claims, characterized in that the foaming surfactant is chosen from anionic surfactants and amphoteric surfactants.

17. Composition according to any one of the preceding claims, characterized in that the foaming surfactant is present in a quantity ranging from 0.5 to 50 % by weight, preferably 2 to 40 % by weight relative to the total weight of the composition.

18. Composition according to any one of Claims 2 to 17, characterized in that water represents from 0.01 to 30 %, advantageously from 2 to 20 %, by weight relative to the total weight of the composition.

19. Composition according to any one of Claims 2 to 18, characterized in that the water is chosen from among Vittel water, Vichy basin waters, Uriage water, Roche Posay water, Bourboule water, Enghien-les-Bains water, Saint Gervais-les-Bains water, Néris-les-Bains water, Allevard-les-Bains water, Digne water, Maizières water, Neyrac-les-Bains water, Lons-le-Saunier water, les Eaux Bonnes water, Rochefort water, Saint Christau water, Fumades water and Tercis-les-bains water, Uriage-les-bains water and Avene water.

20. Composition according to any one of the preceding claims, characterized in that it additionally includes at least one insoluble filler chosen from kaolin, polyethylene powder, polyamide particles and nylon powders.

21. Composition according to any one of the preceding claims, characterized in that it is in the form of a cleaning product, make-up remover, of a hydrating product, of a deep-cleanser or of an exfoliating agent.

**Patentansprüche**

1. Zusammensetzung zur Reinigung der Haut, die das Aussehen eines stabilen, transparenten Gels aufweist oder ein stabiles, transparentes Gel enthält, dadurch gekennzeichnet, daß sie enthält:

   (i) eine Fettphase,
   (ii) mindestens einen $C_{5-7}$-Kohlenhydrat-Fettsäureester,
   (iii) mindestens ein Polyol und
   (iv) mindestens einen schaumbildenden grenzflächenaktiven Stoff.

2. Zusammensetzung nach dem vorhergehenden Anspruch, dadurch gekennzeichnet, daß sie ferner Wasser enthält.

3. Zusammensetzung nach einem der vorhergehenden Ansprüche, dadurch gekennzeichnet, daß das $C_{5-7}$-Kohlen-

hydrat unter Pentosen, Hexosen und Heptosen und deren Alkylglykosid-Derivaten, deren Alkylgruppe 1 bis 6 Kohlenstoffatome aufweist, ausgewählt ist.

4. Zusammensetzung nach einem der vorhergehenden Ansprüche, dadurch gekennzeichnet, daß das Kohlenhydrat das 1-Methylglucosid ist.

5. Zusammensetzung nach einem der vorhergehenden Ansprüche, dadurch gekennzeichet, daß der $C_{5-7}$-Kohlenhydrat-Fettsäureester eine Verbindung ist, die durch Umsetzung einer Fettsäure mit gesättigter oder ungesättigter Kette, die 8 bis 30 Kohlenstoffatome und vorzugsweise 12 bis 22 Kohlenstoffatome aufweist, mit einem Kohlenhydrat hergestellt ist.

6. Zusammensetzung nach einem der vorhergehenden Ansprüche, dadurch gekennzeichnet, daß der $C_{5-7}$-Kohlenhydrat-Fettsäureester alkoxyliert ist.

7. Zusammensetzung nach einem der vorhergehenden Ansprüche, dadurch gekennzeichnet, daß der $C_{5-7}$-Kohlenhydrat-Fettsäureester mit einem oder mehreren Ethylenoxid- oder Propylenoxidfragmenten verethert ist, wobei die gesamten Ethylenoxid- oder Propylenoxid-Substituenten 5 bis 200 Alkylenoxideinheiten ausmachen.

8. Zusammensetzung nach einem der vorhergehenden Ansprüche, dadurch gekennzeichnet, daß der Kohlenhydratester unter den Verbindungen der Formel (I) ausgewählt ist:

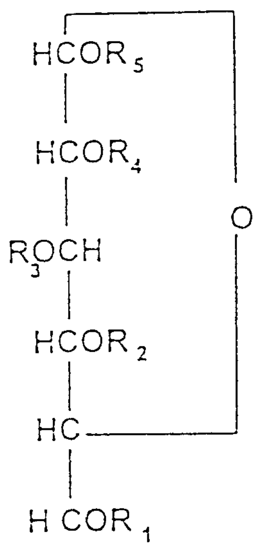

(I),

worin bedeuten:

- $R_1$ eine Gruppe der Formel (III):

$$-OC-R' \qquad (III),$$

  wobei R' unter geradkettigen und verzweigten, gesättigten und ungesättigten $C_{8-30}$-Alkylgruppen und vorzugsweise $C_{12-22}$-Alkylgruppen ausgewählt ist,
- $R_2$, $R_3$, und $R_4$, die identisch oder verschieden sind, eine Gruppe, die ausgewählt ist unter

  - Wasserstoff,
  - einer Gruppe der Formel (II):

$$-(C_nH_{2n}O)_p-R \qquad (II),$$

  worin n = 2 oder 3 ist, p eine ganze Zahl von 2 bis 50 bedeutet und R ein Wasserstoffatom oder eine $C_{1-6}$-Alkylgruppe darstellt,

- eine Gruppe der Formel (III):

$$-OC-R' \qquad (III),$$

wobei R' unter geradkettigen oder verzweigten, gesättigten oder ungesättigten $C_{8-30}$-Alkylgruppen und vorzugsweise $C_{12-22}$-Alkylgruppen ausgewählt ist,

wobei mindestens eine der Gruppen $R_2$, $R_3$, $R_4$ eine Gruppe der Formel (II) ist und die Gesamtzahl $\Sigma p$ der alkoxylierten Reste 5 bis 200 beträgt,

- $R_5$ eine geradkettige oder verzweigte, gesättigte oder ungesättigte $C_{1-6}$-Alkylgruppe.

**9.** Zusammensetzung nach dem vorhergehenden Anspruch, dadurch gekennzeichnet, daß bedeuten:

- $R_5 = CH_3$,
- mindestens zwei Gruppen der Gruppen $R_2$, $R_3$, $R_4$ entsprechen der formel (II),
- $\Sigma p$ liegt im Bereich von 15 bis 150,
- R = H,
- mindestens eine Gruppe der Gruppen $R_1$, $R_2$, $R_3$, $R_4$ entspricht der Formel (III),
- R' ist unter den $C_{16-20}$-Alkylgruppen ausgewählt,
- keine der Gruppen $R_2$, $R_3$, $R_4$ besteht aus Wasserstoff.

**10.** Zusammensetzung nach einem der vorhergehenden Ansprüche, dadurch gekennzeichnet, daß sie 0,5 bis 50 Gew.-% $C_{5-7}$-Kohlenhydratester und vorzugsweise 2 bis 20 Gew.-% $C_{5-7}$-Kohlenhydratester, bezogen auf das Gesamtgewicht der Zusammensetzung, enthält.

**11.** Zusammensetzung nach einem der vorhergehenden Ansprüche, dadurch gekennzeichnet, daß das Polyol unter Ethylenglykol, Propylenglykol, 1,3-Butylenglykol, Dipropylenglykol, Glycerin, Diglycerin, Triglycerin und Tetraglycerin, Glucose, Maltose, Maltit, Saccharose, Fructose, Sorbit, Zuckern, die aus der Zersetzung von Stärke stammen, und deren Gemischen ausgewählt ist.

**12.** Zusammensetzung nach einem der vorhergehenden Ansprüche, dadurch gekennzeichnet, daß sie 0,5 bis 60 Gew.-% Polyol, vorzugsweise 2 bis 40 Gew.-% Polyol und noch bevorzugter 5 bis 30 Gew.-% Polyol, bezogen auf das Gesamtgewicht der Zusammensetzung, enthält.

**13.** Zusammensetzung nach einem der vorhergehenden Ansprüche, dadurch gekennzeichnet, daß die Fettphase ein Öl oder Gemisch von Ölen mit einem Brechungsindex $^{20}n_D \leq 1{,}45$ aufweisen.

**14.** Zusammensetzung nach einem der vorhergehenden Ansprüche, dadurch gekennzeichnet, daß die Fettphase 1 bis 95 Gew.-% und vorzugsweise 20 bis 85 Gew.-% des Gesamtgewichts der Zusammensetzung ausmacht.

**15.** Zusammensetzung nach einem der vorhergehenden Ansprüche, dadurch gekennzeichnet, daß der schaumbildende grenzflächenaktive Stoff unter den grenzflächenaktiven Stoffen ausgewählt ist, die ein Schaumbildungsvermögen aufweisen, das durch eine Schaumhöhe von mehr als 100 mm, vorzugsweise mehr als 120 mm, die nach dem Ross-Miles-Verfahren gemessen wird, für eine Lösung, die 0,1 Gew.-% grenzflächenaktiven Stoff in destilliertem Wasser enthält, bei 25 °C charakterisiert ist.

**16.** Zusammensetzung nach einem der vorhergehenden Ansprüche, dadurch gekennzeichnet, daß der schaumbildende grenzflächenaktive Stoff unter den anionischen grenzflächenaktiven Stoffen und den amphoteren grenzflächenaktiven Stoffen ausgewählt ist.

**17.** Zusammensetzung nach einem der vorhergehenden Ansprüche, dadurch gekennzeichnet, daß der schaumbildende grenzflächenaktive Stoff in einer Menge von 0,5 bis 50 Gew.-%, vorzugsweise 2 bis 40 Gew.-%, bezogen auf das Gesamtgewicht der Zusammensetzung, enthalten ist.

**18.** Zusammensetzung nach einem der Ansprüche 2 bis 17, dadurch gekennzeichnet, daß das Wasser 0,01 bis 30 Gew.-%, vorteilhaft 2 bis 20 Gew.-%, bezogen auf das Gesamtgewicht der Zusammensetzung, ausmacht.

**19.** Zusammensetzung nach einem der Ansprüche 2 bis 18, dadurch gekennzeichnet, daß das Wasser unter Wasser

aus Vittel, Wasser aus dem Vichy-Becken, Wasser aus Uriage, Wasser aus Roche Posay, Wasser aus Bourboule, Wasser aus Enghien-les-Bains, Wasser aus Saint-Gervais-les-Bains, Wasser aus Néris-les-Bains, Wasser aus Allevard-les-Bains, Wasser aus Digne, Wasser aus Maizières, Wasser aus Neyrac-les-Bains, Wasser aus Lons-le-Saunier, den Eaux Bonnes, Wasser aus Rochefort, Wasser aus Saint Christau, Wasser aus Fumades, Wasser aus Tercis-les-bains, Wasser aus Uriage-les-bains und Wasser aus Avenne ausgewählt ist.

**20.** Zusammensetzung nach einem der vorhergehenden Ansprüche, dadurch gekennzeichnet, daß sie ferner mindestens einen unlöslichen Füllstoff enthält, der unter Kaolin, Polyethylenpulver, Polyamidpartikeln und Nylonpulvern ausgewählt ist.

**21.** Zusammensetzung nach einem der vorhergehenden Ansprüche, dadurch gekennzeichnet, daß sie in Form eines reinigenden Produkts eines Produkts zum Abschminken, eines hydratisierenden Produkts, eines abradierend wirkenden Produkts oder eines exfoliativ wirkenden Produkts vorliegt.